(19) Europäisches Patentamt
European Patent Office
Office européen des brevets

(11) Numéro de publication: **0 054 316**
**A1**

# (12) DEMANDE DE BREVET EUROPEEN

(21) Numéro de dépôt: **81201141.9**

(22) Date de dépôt: **15.10.81**

(51) Int. Cl.³: **C 11 B 9/00**
C 07 D 309/30, C 07 D 309/32

(30) Priorité: **09.12.80 CH 9069/80**

(43) Date de publication de la demande:
**23.06.82 Bulletin 82/25**

(84) Etats contractants désignés:
**CH DE FR GB LI NL**

(71) Demandeur: **FIRMENICH SA**
**Case postale 239**
**CH-1211 Genève 8(CH)**

(72) Inventeur: **Fehr, Charles**
**6, chemin Ravoux**
**CH-1290 Versoix/VD(CH)**

(72) Inventeur: **Ohloff, Günther**
**13, chemin de la Chapelle**
**CH-1233 Bernex/GE(CH)**

(74) Mandataire: **Salvadori, Giuseppe, Dr.**
**c/o Firmenich S.A. Case Postale 239**
**CH-1211 Genève 8(CH)**

(54) **Nouveaux composés lactoniques, procédé pour leur préparation et leur utilisation à titre d'ingrédients parfumants ou aromatisants.**

(57) Les composés de formule

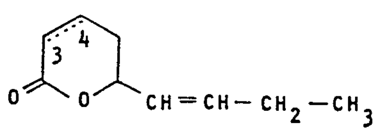

(1)

possédant une simple ou double liaison en position 3 comme indiqué par pointillés, sont utiles à titre d'ingrédients parfumants ou aromatisants, notamment pour développer des notes de type noix, caramel, céleri ou fenugrec.

Leur préparation est effectuée à partir de 2-(but-1-ène-1-yl)-3,4-dihydro-2H-pyranne.

EP 0 054 316 A1

Croydon Printing Company Ltd.

## Nouveaux composés lactoniques, procédé pour leur préparation et leur utilisation à titre d'ingrédients parfumants ou aromatisants

L'invention se rapporte au domaine des parfums et arômes, plus précisément à des composés lactoniques nouveaux utiles à titre d'ingrédients parfumants ou aromatisants.

Elle a notamment pour objet des composés de formule

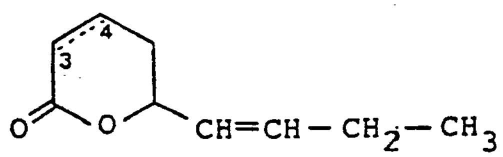

(I)

possédant une simple ou double liaison en position 3 comme indiqué par les pointillés.

Elle a également pour objet un procédé pour la préparation desdits composés, caractérisé en ce que

a) on soumet le 2-(but-1-ène-1-yl)-3,4-dihydro-2H-pyranne à une photooxygénation sensibilisée suivie d'une déshydratation pour obtenir la 6-(but-1-ène-1-yl)-5,6-dihydro-2H-pyranne-2-one ; ou

b) on soumet le 2-(but-1-ène-1-yl)-3,4-dihydro-2H-pyranne à l'action de peroxyde d'hydrogène en présence d'un agent acide suivie d'une déshydratation pour obtenir la 6-(but-1-ène-1-yl)-tétrahydropyranne-2-one.

Elle a en outre pour objet l'utilisation desdits composés de formule (I) à titre d'ingrédients parfumants ou aromatisants.

Elle a finalement pour objet une composition parfumante ou

aromatisante, caractérisée en ce qu'elle contient un composé de formule (I) à titre d'ingrédient organoleptiquement actif.

La formule (I) définie ci-dessus sert à désigner des composés lactoniques nouveaux dans lesquels la double liaison de la chaîne latérale peut être de configuration cis ou trans, à savoir la 6-(cis-but-1-ène-1-yl)-5,6-dihydro-2H-pyranne-2-one, la 6-(trans-but-1-ène-1-yl)-5,6-dihydro-2H-pyranne-2-one, la 6-(cis-but-1-ène-1-yl)-tétrahydropyranne-2-one et la 6-(trans-but-1-ène-1-yl)-tétrahydropyranne-2-one.

Malgré l'abondance de corps odoriférants lactoniques connus [voir à ce sujet S. Arctander, Perfume and Flavor Chemicals, Montclair N. J. 1969, Sections 416, 828, 829, 1102, 1103, 1504, 1590, 1648], un enrichissement constant de la palette du parfumeur demeure nécessaire. La recherche de structures nouvelles et l'élaboration d'effets organoleptiques originaux sont en fait indispensables au progrès de l'industrie des parfums et arômes : l'invention permet d'y contribuer efficacement.

Dans le domaine des parfums, les composés de formule (I) se distinguent par une odeur de type coumariné enrichie de tonalités originales rappelant le céleri, la noix, le jasmin et le fenugrec. De ce fait, ils se prêtent avantageusement à la préparation de parfums fort divers, notamment de type fruité, épicé, chypre, boisé, jasmin, rose ou fougère auxquels ils contribuent à donner richesse et harmonie. La 6-(but-1-ène-1-yl)-5,6-dihydro-2H-pyranne-2-one possède entre autres la particularité de développer une note épicée de type fenugrec qui, tout en ne dominant pas, contribue à donner une certaine originalité à la composition de base à laquelle on l'a incorporée.

Les composés de formule (I) peuvent être tout aussi avantageusement utilisés pour le parfumage de produits aussi variés que savons, détergents, laits ou crèmes de beauté, lotions, shampoings ou produits d'entretien par exemple.

Des effets olfactifs tels que ceux définis ci-dessus peuvent être obtenus par l'emploi d'un composé de formule (I) à titre d'ingrédient parfumant unique, sous forme de solution dans les solvants usuels tels que alcool éthylique, phtalate diéthylique ou dipropylène-glycol par exemple, ou encore conjointement à d'autres ingrédients odoriférants, dans ce cas sous forme de base, composition ou coeur pour parfums. Lors de la préparation de compositions parfumantes par exemple, des effets olfactifs intéressants s'obtiennent par l'emploi de quantités de l'ordre de 0,1% déjà. Selon les effets particuliers recherchés, ces quantités peuvent aller jusqu'à 1, voire 5% environ par rapport au poids de la composition considérée.

Dans le domaine des arômes, les composés de formule (I) se caractérisent par une note gustative que l'on peut qualifier de légèrement fleurie, coumarinée, verte, herbale et fraîche, accompagnée d'une tonalité de type noix, céleri et livèche. Ils trouvent de ce fait un emploi fort avantageux dans la préparation d'arômes artificiels variés, tels des arômes de caramel, noix ou menthe par exemple dans lesquels ils peuvent développer des effets gustatifs analogues à ceux de la coumarine.

Les composés de formule (I) peuvent être également utilisés pour l'aromatisation d'aliments divers, de boissons, de préparations pharmaceutiques ou du tabac. Lors de l'aromatisation d'aliments ou boissons par exemple, des effets gustatifs intéressants peuvent être obtenus par l'emploi de quantités comprises entre 1 et 50 ppm (parties par millions) environ, de préférence voisines de 20 ppm environ par rapport au poids de la matière ainsi aromatisée.

Afin de produire l'effet aromatisant attendu, les composés (I) peuvent être directement ajoutés à l'aliment ou boisson considéré. On les utilise cependant de préférence sous forme de solution dans un solvant comestible usuel tel le dipropylène-glycol ou la triacétine par exemple, ou encore adsorbés sur un support solide tel que la gomme arabique ou la dextrine par exemple.

Les composés de formule (I) sont des composés nouveaux ; selon le procédé de l'invention, ils sont obtenus à partir de 2-(but-1-ène-1-yl)-3,4-dihydro-2H-pyranne de formule

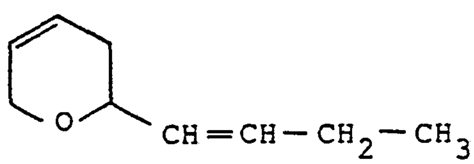

(II)

à titre de produit de départ.

En effet, selon l'invention, la 6-(but-1-ène-1-yl)-5,6-dihydro-2H-pyranne-2-one (Ia) peut être préparée à partir du composé de formule (II) ci-dessus, par une photooxygénation sensibilisée suivie de la déshydratation de l'hydroperoxyde intermédiaire formé. La photooxygénation sensibilisée du dihydropyranne correspondant s'effectue selon les conditions usuelles en chimie organique, par exemple selon les méthodes répertoriées à ce propos dans Organic Reactions, Vol. 20, p. 245 (1973).

Selon l'invention également, la 6-(but-1-ène-1-yl)-tétrahydro-

pyranne-2-one (Ib) peut être obtenue à partir du composé de formule (II) défini précédemment par traitement de ce dernier au moyen de peroxyde d'hydrogène, en présence d'un agent acide, suivi de la déshydratation de l'hydroperoxyde intermédiaire formé.Ces deux traitements successifs s'effe tuent selon les techniques usuelles [voir par exemple H. O. House, Modern Synthetic Reactions, W. A. Benjamin Inc., 1972, p. 292 et suivantes].

La préparation desdites lactones sera donnée par le détail dans les exemples qui suivent et peut être schématisée comme suit :

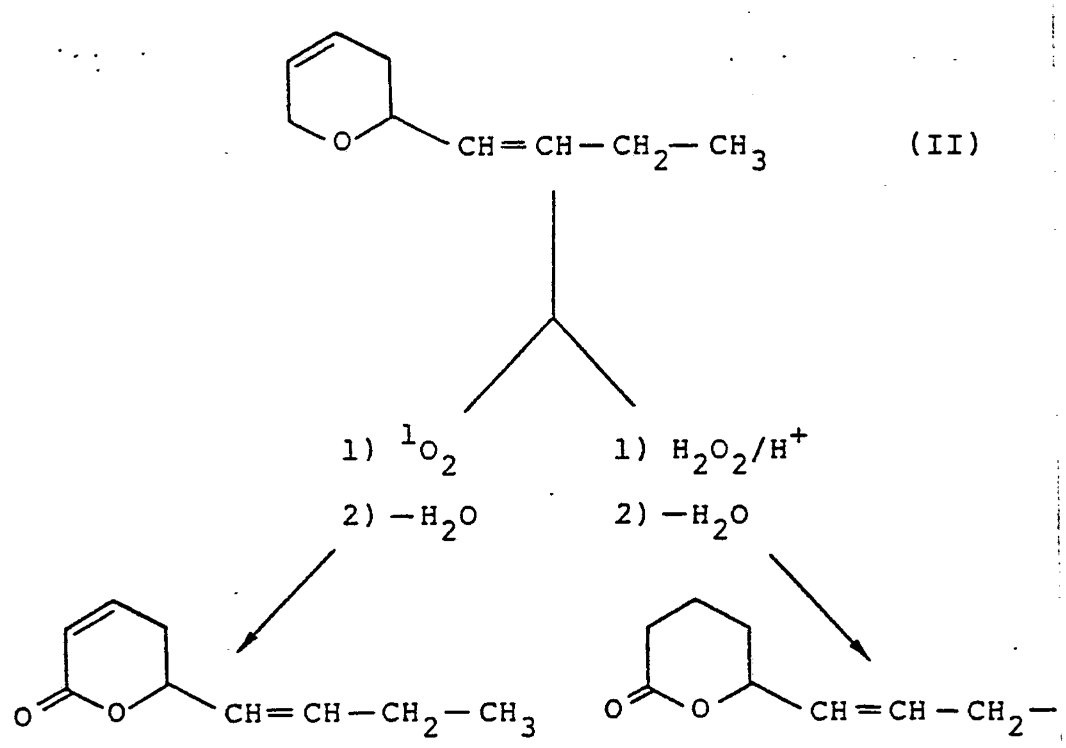


(II)

(Ia) (Ib)

La configuration de la double liaison de la chaîne latérale des composés de formule (I) ainsi obtenus est déterminée par celle de la chaîne latérale du composé de départ de formule (II). Ainsi, le 2-(cis-but-1-ène-1-yl)-3,4-dihydro-2H-pyranne donnera-t-il naissance à la 6-(cis-but-1-ène-1-yl)-5,6-dihydro-2H-pyranne-2-one et à la 6-(cis-but-1-ène-1-yl)-tétrahydrop ranne-2-one. De façon identique, un mélange de 2-(cis et trans-but-1-ène-1-yl)-3,4-dihydro-2H-pyranne tel qu'il peut résulter de la synthèse conduira-t-il à un mélange isomérique correspondant du composé de formule (Ia) ou (Ib).

Selon l'invention, les composés de formule (I) peuvent être utilisé sous forme isomérique pure ou sous forme de mélanges d'isomères ; pour des raisons d'ordre pratique et économique cependant, on utilisera lesdits

composés tels que directement issus du procédé de l'invention.

Le 2-(but-1-ène-1-yl)-3,4-dihydro-2H-pyranne (II) utilisé à titre de produit de départ selon le procédé de l'invention, est également un produit nouveau. Il peut être obtenu par exemple à partir du dimère de l'acroléine, après traitement de ce dernier au moyen de bromure de triphényl-propyl-phosphonium dans les conditions d'une réaction dite de Wittig.

L'invention sera illustrée de façon plus détaillée à l'aide des exemples ci-après (températures en degrés centigrades).

Exemple 1

Préparation de 6-(but-1-ène-1-yl)-5,6-dihydro-2H-pyranne-2-one

A. (Préparation du produit de départ).3,27 g (75 mmole) d'une dispersion de NaH à 55% dans l'huile minérale ont été premièrement lavés au moyen d'éther de pétrole 30-50 (3 x 20 ml), puis chauffés durant 45 min à 80° en présence de 90 ml de diméthyl-sulfoxyde. Après refroidissement du mélange à 20°, on a ajouté 19,3 g (50 mmole) de bromure de triphényl-propyl-phosphonium, par petites portions, l'agitation du mélange obtenu étant poursuivie durant 1 nuit à température ambiante. 12,32 g (110 mmole) d'acroléine-dimère ont ensuite été ajoutés au mélange refroidi (0°), à une cadence telle que la température de réaction reste inférieure à 35°. Après addition, le mélange a été agité durant 15 min, traité au moyen d'éther de pétrole 30-50 (250 ml) et d'eau (200 ml), puis filtré. Après lavage du précipité recueilli (triphénylphosphine-oxyde) au moyen d'éther de pétrole 30-50 (100 ml), lavage du filtrat avec $H_2O$, puis de la saumure, la phase organique a été séchée sur $Na_2SO_4$ et finalement concentrée sous pression réduite (20°/15,96 x $10^2$ Pascals). Après distillation du résidu dans un tube à boules (température du bain 100-140°/6,65 x $10^2$ Pascals), on a isolé 5,13 g (rendement 74%) de 2-(but-1-ène-1-yl)-3,4-dihydro-2H-pyranne.

IR : 2995, 1640, 1460, 1390, 1375, 1345, 1235, 1215, 1060, 985, 965, 915, 895 $cm^{-1}$ ;

RMN (60 MHz) : 1,00 (3H, t, J=7,5 Hz) ; 1,60-2,35 (6H, m) ; 4,63 (1H, m), 5,2-5,8 (3H, m) ; 6,35 (1H, d, J=6,5 Hz) $\delta$ ppm ;

SM : $M^+$ = 138(12) ; m/e : 109(18), 82(43), 81(18), 79(14), 67(100), 55(14), 41(31), 39(23), 27(31).

Selon l'analyse par chromatographie en phase gazeuse (colonne

de silicone SE 30 à 5% - 1,60 m - 60°), le produit identifié ci-dessus consiste en un mélange 84:16 d'isomères cis/trans.

Temps de rétention : isomère cis 4,5 min
isomère trans 5,4 min

B. 2,0 g (14,5 mmole) du produit obtenu ci-dessus ont été soumis à une photooxygénation sensibilisée en présence de toluène (80 ml) et de m-tétraphénylporphine (100 mg). Après consommation de 400 ml environ d'oxygène, le mélange résultant a été traité au moyen de 2 ml de triéthylamine et 2 ml d'anhydride acétique jusqu'à obtention d'un test de peroxydes négatif. Après concentration, le mélange obtenu a été extrait à l'éther et la phase organique lavée avec $H_2O$, $NaHCO_3$ à 5% dans $H_2O$, puis de la saumure jusqu'à neutralité et finalement évaporée. Après distillation du résidu (température du bain 110-140°/6,65 Pascals), on a isolé 1,25 g (rendement 57%) de 6-(but-1-ène-1-yl)-5,6-dihydro-2H-pyranne-2-one.

IR : 2995, 1705, 1450, 1405, 1370, 1230, 1140, 1125, 1035, 1005, 950, 875, 855 $cm^{-1}$ ;

RMN (60 MHz) : 1,00 (3H, t, J=7,5 Hz) ; 1,90-2,60 (4H, m) ; 5,25 (1H, m) ; 5,56 (2H, m) ; 5,97 (1H, dt, J=2 et 10 Hz) ; 6,92 (1H, dt, J=4,5 et 10 Hz) δ ppm ;

SM : $M^+$ : 152(0,1) ; m/e : 121(32), 110(10), 69(14), 68(100), 55(11), 44(13), 41(20), 40(16), 39(25), 28(19), 27(13).

Selon l'analyse par chromatographie en phase gazeuse (colonne de silicone SE 30 à 5% - 1,60 m - 160°), le produit identifié ci-dessus consiste en un mélange d'isomères cis/trans.

Temps de rétention : isomère cis 5,3 min
isomère trans 6,5 min

## Exemple 2

Préparation de 6-(but-1-ène-1-yl)-tétrahydropyranne-2-one

2,0 g (15,2 mmole) de 2-(but-1-ène-1-yl)-3,4-dihydro-2H-pyranne - voir Exemple 1 · 10 ml de tétrahydrofuranne et 3,48 g (35,8 mmole) de $H_2O_2$ à 35% ont été agités pendant 24 h à température ambiante, en présence de 100 mg environ de $H_2SO_4$ à 98%. Le mélange de réaction a ensuite été versé dans une solution aqueuse saturée de sulfate d'ammonium, extrait au chlorure de méthylène (3 x 50 ml) et la phase organique finalement lavée au moyen de $NaHCO_3$

saturé, puis de saumure, séchée sur $Na_2SO_4$ et filtrée. Le filtrat ainsi obtenu a ensuite été traité au moyen de 2 ml de triéthylamine et 2 ml d'anhydride acétique jusqu'à obtention d'un test de peroxydes négatif. Après concentration, le mélange a été extrait à l'éther et la phase organique lavée avec $NaHCO_3$ à 5% dans $H_2O$ puis de la saumure jusqu'à neutralité et finalement évaporée. Le résidu brut ainsi obtenu a été finalement purifié par chromatographie sur gel de silice (éluant : cyclohexane/acétate d'éthyle 19:1) pour donner 1,0 g (rendement 45%) de 6-(but-1-ène-1-yl)-tétrahydropyranne-2-one.

IR : 2980, 1730, 1165, 1440, 1365, 1345, 1235, 1185, 1160, 1030, 890 $cm^{-1}$ ;
RMN (60 MHz) : 1,00 (3H, t, J=7,5 Hz) ; 1,70-2,35 (6H, m) ; 2,53 (2H, m) ; 5,10 (1H, m) ; 5,50 (2H, m) δ ppm ;
SM : $M^+$ : 154(0,5) ; m/e : 125(35), 97(41), 94(35), 79(22), 70(41), 67(53), 55(61), 43(22), 42(100), 39(32), 27(35).

Selon l'analyse par chromatographie en phase gazeuse (colonne de silicone SE 30 à 5% - 1,60 m - 100°), le produit identifié ci-dessus consiste en un mélange 84:16 d'isomères cis/trans.

Temps de rétention : isomère cis 5,7 min
isomère trans 6,1 min

## Exemple 3

On a préparé une composition parfumante de base de type "chypre" en mélangeant les ingrédients suivants (parties en poids) :

| Ingrédients | Parties en poids |
|---|---|
| Essence de patchouli | 150 |
| Triméthyl-cyclododécatriène-époxyde | 100 |
| HEDIONE ®1) | 65 |
| Essence de mandarine | 50 |
| Acétate de benzyle | 50 |
| IRALIA ®1) | 50 |
| Aldéhyde α-hexyl-cinnamique | 50 |
| Base CYCLOSIA ®1) | 50 |
| Essence de santal oriental | 50 |
| Absolu de jasmin synthétique | 50 |

| | |
|---|---|
| Essence de bergamote synthétique | 50 |
| Musc cétone | 40 |
| Acétate de vétivéryle | 35 |
| Essence de sauge sclarée | 30 |
| Absolu de mousse de chêne à 50% * | 25 |
| EXALTEX ®1) | 20 |
| Aldéhyde cuminique à 10% * | 20 |
| Essence de clous de girofle | 20 |
| Essence d'estragon | 20 |
| Acétate de styrallyle | 15 |
| Aldéhyde undécylénique à 10% * | 15 |
| 2,5-Diméthyl-résorcinate de méthyle | 10 |
| FIXATEUR 404 1) | 5 |
| Aldéhyde décylique à 10% * | 5 |
| Aldéhyde dodécylique à 10% * | 5 |
| γ-Undécalactone | 5 |
| Isobutyrate de diméthyl-benzyl-carbinyle | 5 |
| Total | 990 |

* dans le phtalate diéthylique

1) origine : FIRMENICH SA, Genève - Suisse

En ajoutant à 99 g de la composition de base ci-dessus 1 g d'une solution à 10% * de 6-(but-1-ène-1-yl)-5,6-dihydro-2H-pyranne-2-one (mélange isomérique cis/trans ; voir Exemple 1), on obtient une nouvelle composition parfumante dont le côté fruité-chypré est devenu plus riche et plus harmonieux.

En remplaçant dans l'exemple ci-dessus la 6-(but-1-ène-1-yl)-5,6-dihydro-2H-pyranne-2-one par une quantité identique de 6-(but-1-ène-1-yl)-tétrahydropyranne-2-one (mélange isomérique cis/trans ; voir Example 2), on obtient un effet analogue quoique moins marqué.

Exemple 4

On a préparé une composition aromatisante de base de type vanille-caramel en mélangeant les ingrédient suivants (parties en poids) :

| | |
|---|---|
| Maltol | 15 |
| Vanilline | 200 |
| Ethylvanilline | 50 |

| | |
|---|---|
| Alcool anisique | 10 |
| Acétoïne | 5 |
| Ether de Rhum | 20 |
| Propylène-glycol | 700 |
| Total | 1000 |

En utilisant 100 g de la composition indiquée ci-dessus, on a préparé les deux nouvelles compositions définies ci-après.

| | A ("témoin") | B ("test") |
|---|---|---|
| Composition de base | 100 | 100 |
| 6-(But-1-ène-1-yl)-5,6-dihydro-2H-pyranne-2-one 1) à 10% dans l'éthanol à 95% | - | 20 |
| Ethanol à 95% | 900 | 880 |
| | 1000 | 1000 |

1) mélange isomérique cis/trans (voir Exemple 1)

Les compositions A et B ainsi préparées, diluées dans de l'eau de source à raison de 0,1%, ont été évaluées par un groupe d'experts qui était appelé à se prononcer sur leurs propriétés gustatives.

Les commentaires formulés peuvent se résumer ainsi :

Composition A : note vanille
Composition B : note vanille-caramel avec un arrière goût légèrement coumariné.

En remplaçant dans l'exemple ci-dessus la 6-(but-1-ène-1-yl)-5,6-dihydro-2H-pyranne-2-one par une quantité identique de 6-(but-1-ène-1-yl)-tétrahydropyranne-2-one (mélange isomérique cis/trans ; voir Example 2), on obtient un effet analogue quoique moins marqué.

## Exemple 5

0,5 g d'une solution à 1% de 6-(but-1-ène-1-yl)-5,6-dihydro-2H-pyranne-2-one dans l'éthanol à 95% ont été dispersés sur 100 g d'un mélange de tabacs de type "american blend". Le tabac aromatisé ainsi a ensuite été utilisé pour la manufacture de cigarettes "test" dont la fumée a été

soumise à une évaluation organoleptique, après comparaison de celle-ci avec la fumée de cigarettes "témoin", non aromatisée, dont le tabac avait été préalablement traité par de l'éthanol à 95%.

Le groupe d'experts requis a déclaré que la fumée de cigarettes "test" possédait une note plus douce et agréable et un caractère coumariné.

En remplaçant dans l'exemple ci-dessus la 6-(but-1-ène-1-yl)-5,6-dihydro-2H-pyranne-2-one par une quantité identique de 6-(but-1-ène-1-yl)-tétrahydropyranne-2-one (mélange isomérique cis/trans ; voir Exemple 2), on obtient un effet analogue quoique moins marqué.

REVENDICATIONS

1. Composés de formule

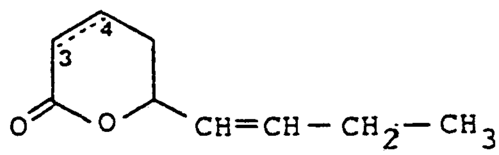

(I)

possédant une simple ou double liaison en position 3 comme indiqué par les pointillés.

2. 6-(cis-But-1-ène-1-yl)-5,6-dihydro-2H-pyranne-2-one.

3. 6-(trans-But-1-ène-1-yl)-5,6-dihydro-2H-pyranne-2-one.

4. 6-(cis-But-1-ène-1-yl)-tétrahydropyranne-2-one.

5. 6-(trans-But-1-ène-1-yl)-tétrahydropyranne-2-one.

6. Procédé pour la préparation d'un composé de formule (I) suivant la revendication 1, caractérisé en ce que

a) on soumet le 2-(but-1-ène-1-yl)-3,4-dihydro-2H-pyranne à une photooxygénation sensibilisée suivie d'une déshydratation pour obtenir la 6-(but-1-ène-1-yl)-5,6-dihydro-2H-pyranne-2-one ; ou

b) on soumet le 2-(but-1-ène-1-yl)-3,4-dihydro-2H-pyranne à l'action de peroxyde d'hydrogène en présence d'un agent acide suivie d'une déshydratation pour obtenir la 6-(but-1-ène-1-yl)-tétrahydropyranne-2-one.

7. Utilisation à titre d'ingrédient parfumant ou aromatisant d'un des composés suivant la revendication 1.

8. Composition parfumante ou aromatisante, caractérisée en ce qu'elle contient un des composés suivant la revendication 1 à titre d'ingrédient organoleptiquement actif.

0054316

Office européen des brevets

**RAPPORT DE RECHERCHE EUROPEENNE**

Numéro de la demande

EP 81 20 1141

| DOCUMENTS CONSIDERES COMME PERTINENTS | | | CLASSEMENT DE LA DEMANDE (Int. Cl. 3) |
|---|---|---|---|
| Catégorie | Citation du document avec indication, en cas de besoin, des parties pertinentes | Revendication concernée | |
| A | FR - A - 2 385 711 (POLAK'S FRUTAL WORKS)<br>* Pages 1,3-5,6 exemple 3, pages 9,10 *<br>-- | 1,7,8 | C 11 B 9/00<br>C 07 D 309/30<br>309/32 |
| A | FR - A - 2 385 394 (BEHEER)<br>* Pages 1,3-6,9,10 *<br>-- | 1,7,8 | |
| A | CHEMICAL ABSTRACTS, vol. 75, 1971, page 414, ref. 76114j<br>Columbus, Ohio, US<br>A.IJIMA et al.: "Synthesis of δ-lactones. I. 6-Alkyl, 6-alkenyl, or 6-aralkyl δ-lactones from dihydroresorcinol"<br>& Chem. Pharm. Bull. 1971, 19(5), 1053-5<br>* Résumé *<br>-- | 1,4,5 | DOMAINES TECHNIQUES RECHERCHES (Int. Cl. 3)<br>C 07 D 309/00 |
| A | JOURNAL OF THE AMERICAN CHEMICAL SOCIETY, (1980), 102, pages 6889-6891<br>Washington, US<br>H. MACKE et al.: "Claisen Rearrangements of Lactonic (Silyl) Enolates: A New Route of Functionalized Cycloalkenes"<br>* Pages 6889-90, table I(5e) *<br>---- | 1,4,5 | CATEGORIE DES DOCUMENTS CITES<br>X: particulièrement pertinent à lui seul<br>Y: particulièrement pertinent en combinaison avec un autre document de la même catégorie<br>A: arrière-plan technologique<br>O: divulgation non-écrite<br>P: document intercalaire<br>T: théorie ou principe à la base de l'invention<br>E: document de brevet antérieur, mais publié à la date de dépôt ou après cette date<br>D: cité dans la demande<br>L: cité pour d'autres raisons |
| X | Le présent rapport de recherche a été établi pour toutes les revendications | | &: membre de la même famille, document correspondant |

| Lieu de la recherche | Date d'achèvement de la recherche | Examinateur |
|---|---|---|
| La Haye | 15-03-1982 | FRANCOIS |